# EUROPEAN PATENT APPLICATION

(11) **EP 1 507 004 A1**
(43) Date of publication of application: **16.02.2005**
(21) Application number: 03018451.9
(22) Date of filing: 14.08.2003
(51) Int. Cl.: C12N 15/11, A61K 31/711, G01N 33/53

(54) **Method to inhibit the propagation of an undesired cell population**

(71) Applicant: DKFZ Deutsches Krebsforschungszentrum, 69120 Heidelberg (DE); INSERM, The French Institute of Health and Medical Research, 75654 Paris Cédex 13 (FR)
(72) Inventor: Cziepluch, Celina, 69115 Heidelberg (DE); Rommelaere, Jean, 69118 Heidelberg (DE); Winnefeld, Marc, 69117 Heidelberg (DE)
(74) Representative: Huhn, Michael, Dr.

(57) **Abstract**

Disclosed is a method for inhibiting the propagation of an undesired cell population, the method comprising introducing into a cell an antagonist of SGT, or of a functional variant thereof, said antagonist depleting SGT, a functional variant thereof, in a cell of said cell population, and verifying the reduced proliferation and/or cell death in SGT depleted cell populations; disclosed is further the use of a SGT antagonist for the manufacture of a medicament for the treatment of diseases which are caused by the propagation of an undesired cell population.

SEQUENCE LISTING

<110> Deutsches Krebsforschungszentrum

<120> Method to inhibit the propagation of an undesired cell population

<130> DK62328EP

<160> 4

<170> PatentIn version 3.1

<210> 1
<211> 313
<212> PRT
<213> Homo sapiens

<400> 1

Met Asp Asn Lys Lys Arg Leu Ala Tyr Ala Ile Ile Gln Phe Leu His
1               5                   10                  15

Asp Gln Leu Arg His Gly Gly Leu Ser Ser Asp Ala Gln Glu Ser Leu
            20                  25                  30

Glu Val Ala Ile Gln Cys Leu Glu Thr Ala Phe Gly Val Thr Val Glu
        35                  40                  45

Asp Ser Asp Leu Ala Leu Pro Gln Thr Leu Pro Glu Ile Phe Glu Ala
    50                  55                  60

Ala Ala Thr Gly Lys Glu Met Pro Gln Asp Leu Arg Ser Pro Ala Arg
65                  70                  75                  80

Thr Pro Pro Ser Glu Glu Asp Ser Ala Glu Ala Glu Arg Leu Lys Thr
                85                  90                  95

Glu Gly Asn Glu Gln Met Lys Val Glu Asn Phe Glu Ala Ala Val His
            100                 105                 110

Phe Tyr Gly Lys Ala Ile Glu Leu Asn Pro Ala Asn Ala Val Tyr Phe
        115                 120                 125

Cys Asn Arg Ala Ala Ala Tyr Ser Lys Leu Gly Asn Tyr Ala Gly Ala
    130                 135                 140

Val Gln Asp Cys Glu Arg Ala Ile Cys Ile Asp Pro Ala Tyr Ser Lys
145                 150                 155                 160

Ala Tyr Gly Arg Met Gly Leu Ala Leu Ser Ser Leu Asn Lys His Val
                165                 170                 175

Glu Ala Val Ala Tyr Tyr Lys Lys Ala Leu Glu Leu Asp Pro Asp Asn
            180                 185                 190

Glu Thr Tyr Lys Ser Asn Leu Lys Ile Ala Glu Leu Lys Leu Arg Glu
        195                 200                 205

Ala Pro Ser Pro Thr Gly Gly Val Gly Ser Phe Asp Ile Ala Gly Leu
    210                 215                 220

Leu Asn Asn Pro Gly Phe Met Ser Met Ala Ser Asn Leu Met Asn Asn
225                 230                 235                 240

Pro Gln Ile Gln Gln Leu Met Ser Gly Met Ile Ser Gly Gly Asn Asn
                245                 250                 255

Pro Leu Gly Thr Pro Gly Thr Ser Pro Ser Gln Asn Asp Leu Ala Ser
            260                 265                 270

Leu Ile Gln Ala Gly Gln Gln Phe Ala Gln Gln Met Gln Gln Gln Asn
        275                 280                 285

Pro Glu Leu Ile Glu Gln Leu Arg Ser Gln Ile Arg Ser Arg Thr Pro
    290                 295                 300

Ser Ala Ser Asn Asp Asp Gln Gln Glu
305                 310

<210>  2

<211>  2218

<212>  DNA

<213>  Homo sapiens


<400>  2
   tcgacggtcg cctgagaggt atcacctctt ctgggctcaa gatggacaac aagaagcgcc      60
   tggcctacgc catcatccag ttcctgcatg accagctccg gcacgggggc ctctcgtccg     120
   atgctcagga gagcttggaa gtcgccatcc agtgcctgga gactgcgttt ggggtgacgg     180
   tagaagacag tgaccttgcg ctccctcaga ctctgccgga gatatttgaa gcggctgcca     240
   cgggcaagga gatgccgcag gacctgagga gccccgcgcg aaccccgcct tccgaggagg     300
   actcagcaga ggcagagcgc ctcaaaaccg aaggaaacga gcagatgaaa gtggaaaact 360
   ttgaagctgc cgtgcatttc tacggaaaag ccatcgagct caacccagcc aacgccgtct 420
   atttctgcaa cagagccgca gcctacagca aactcggcaa ctacgcaggc gcggtgcagg 480
   actgtgagcg ggccatctgc attgacccgg cctacagcaa ggcctacggc aggatgggcc 540
   tggcgctctc cagcctcaac aagcacgtgg aggccgtggc ttactacaag aaggctctgg 600
   agctggaccc cgacaacgag acatacaagt ccaacctcaa gatagcggag ctgaagctgc 660
   gggaggcccc cagccccacg ggaggcgtgg gcagcttcga catcgccggc ctgctgaaca 720
   accctggctt catgagcatg gcttcgaacc taatgaacaa tccccagatt cagcagctca 780
   tgtccggcat gatttcgggt ggcaacaacc ccttgggaac tcccggcacc agcccctcgc 840
   agaacgacct ggccagcctc atccaggcgg gccagcagtt tgcccagcag atgcagcagc 900
   agaacccaga gttgatagag cagctcagga gccagatccg gagtcggacg cccagcgcca 960
   gcaacgacga ccagcaggag tgacgctgcc tgctcccggt gtgaccgcgt ccttccctgg 1020
   ccgacccgaa ggaagccttc tggttgtctg ccacttcctc ctgttggact gcctgagaga 1080
   ggggaagaga gagacctcgg acctgcatgt caagatggat tttccccttt tatctctgcc 1140
   ctcctccact ccctttttgt aactccctta cagcccccag acccttcttg aaacgagagc 1200
   cagcaagctg agcacagacc agcagcgacc tcccttccag cccccagaaa gctcggtcac 1260
   ttgagtgttt tctagaatcc tggggtgctc ccgggccgct ctcagagaag tggcaggttt 1320
   cacgttcagc cgtgtggcgg atcgtgtggc ttccaaagcc ttttacagcc cccgcccccc 1380
   atcccgtggt ctgtctgcag gaactctccc gtctgtgaga agcctctttc cgagtcgacc 1440
   tcccggccac cccggccctg tgcctgctcg gaagagctca ctgccagctg cggcctgggc 1500
   accgcgggcc atgtgtgttt gcatgaggaa ctctttagtg gcagacacct aagagacggc 1560
   tgcggtcacc ccacgcctcc gcggctcagg agccgtcctg ggtgcatagg accagtttct 1620
   gtgacttttc tccagttggg catgttgaca gacatgtttc ccctcctccc accctcattt 1680
   tctggtcctc gcgactgaga gccaggggcg acatcatgac cttctgtccc ggccgcctta 1740
   gccccgggca cagggaaggc agctgggccg tttctgtctg tgtcccatcc tgctgtcctt 1800
   ctgtcctgga tgtttcatgc ccggggcccc ccagggaagc ttacccctcc tgtgctgggt 1860
   ggaggccacg ggacacctca ggtgccaccc accttggccc taaaacagcc accaggaaag 1920
   cagccagaga gccggacagc aggcagcctg tctgggttcc tgaggcctgg gggtggcaga 1980
   cgagcccacg gcgccgtggt cccagcagca gggttgtcag tcggagcatc ctggggctcc 2040
   ctggctcctg gccgtctgtg aggtaggcgc agtaccgtgt atcgtaggta gcagtaggaa 2100
   cgggggccgc cgcggccctg cagccgctca tggcggtgag gtgtgtgcca agcccacccg 2160
   gggtgcaggg cgtgacgtgt ggggaataaa taggcgttgt gacctcaaaa aaaaaaaa 2218

<210> 3

<211>  21

<212>  DNA

<213>  Homo sapiens

<400>  3
aacttgaagc tgccgtggat t                                                21

<210>  4

<211>  23

<212>  DNA

<213>  Homo sapiens

<400>  4
aagcacgtgg aggccgtggc tta                                              23

## Description

The present invention refers to a method to eliminate an undesired cell population, particularly aberrantly growing tumor cells, by specifically inactivating or depleting the human Small Glutamine-rich Tetratricopeptide repeat-containing protein (hSGT).

Cell division and proliferation is a normal ongoing process in all living organisms and involves numerous factors and signals that are delicately balanced to maintain regular cellular cycles. The general process of cell division consists of two sequential processes: nuclear division (mitosis), and cytoplasmic division (cytokinesis). Because organisms are continually growing and replacing cells, cellular proliferation is vital to the normal functioning of almost all biological processes. Whether or not mammalian cells will grow and divide is determined by a variety of feedback control mechanisms, which include the availability of space in which a cell can grow, and the secretion of specific stimulatory and inhibitory factors in the immediate environment. When normal cellular proliferation is disturbed or somehow disrupted, the results can affect an array of biological functions. Disruption of proliferation could be due to a myriad of factors such as the absence or overabundance of various signaling chemicals or presence of altered environments. Disorders characterized by abnormal cellular proliferation include cancer, abnormal development of embryos, improper formation of the corpus luteum, difficulty in wound healing as well as malfunctioning of inflammatory and immune responses.

Abnormally proliferating cancer cells exhibit a number of properties that make them dangerous to the host, often including an ability to invade other tissues. One of the defining features of cancer cells is that they respond abnormally to control mechanisms that regulate the division of normal cells and continue to divide in a relatively uncontrolled fashion until they kill the host. The effective cure of patients, though, is often difficult since many tumor cells develop a resistance to radiation therapy or cytostatic drugs used for chemotherapy.

The described phenotype, also termed drug resistance, involves a variety of strategies that tumor cells use to evade the cytostatic or cytolytic effects of anticancer drugs. Mechanisms for drug resistance include modifications in detoxification and DNA repair pathways, changes in cellular sites of drug sequestration, decreases in drug-target affinity, synthesis of specific drug inhibitors within cells, and accelerated removal or secretion of drugs. More recently, cellular drug resistance has been associated with alterations in the so-called programmed cell death, a process known as apoptosis. Thus, many methods of the prior art which are employed to solve the problem of successfully circumventing drug-resistance rely on compounds or methods which directly affect, i.e. enhance, various steps in apoptosis. Other approaches include, for example, the up- or down-regulation of distinct genes which have demonstrated to be deregulated in drug-resistant tumor cells.

In view of the increasing number in cancer cases, any approach to improve current treatment methods is of important ethic, social and economic value. Thus, a constant search for novel and alternative methods and compounds which eliminate abnormal cell growth as it is the case with cancer is desirable. Furthermore, a variety of other circumstances like e.g. inflammatory diseases or viral infections can make it necessary to eliminate cells which are infected by various microorganisms.

Thus, the problem underlying the present invention is to find a reliable, pan-acting target, compound or method which can be used to eliminate cells which are abnormally growing, inflamed, infected or otherwise altered, making it desirable to eliminate the affected cell population.

The inventors have now found that the Small Glutamine-rich Tetratricopeptide repeat (TPR)-containing (SGT) protein is required for proper progress through cell division and, therefore, represents a novel target suitable for manipulating cell propagation.

Human SGT (hSGT) was initially discovered as cellular binding partner for the non-structural protein of autonomous parvovirus H-1 (Cziepluch et al., 1998, J. Virol., Vol.72, pages 4149-5156; Kordes et al., 1998, Genomics, Vol. 52, pages 90-94) and as partner for the Human Immune-deficiency Virus-encoded Vpu (viral protein "U"), termed Ubp - for "U binding protein". From the latter, a potential use for SGT/Ubp in therapy against HIV infections was proposed (see US-B 6,392,014). Furthermore, SGT may be a useful target in the treatment of Alzheimer's Disease as deduced from the observation, that C.elegans strains expressing beta-amyloid peptide (A-beta), a primary component of senile plaques characteristic for Alzheimer's disease, can be protected from A-beta-induced toxicity by inhibiting SGT expression (Fonte et al., 2002, Proc.Natl.Acad.Sci., Vol. 99, pages 9439-9444)

The present invention solves the problem of eliminating undesired cell populations by depleting hSGT. Cell populations, the hSGT protein levels of which have been strongly depleted, displayed reduced proliferation. Additionally, a significant proportion of cells from these hSGT knock down populations displayed an arrest in mitosis frequently followed by cell death. Thus, the present invention provides a novel method to directionally inhibit the proliferation and propagation of abnormally growing cells, like tumor cells, by inactivating or depleting hSGT.

Therefore, a first aspect of the present invention is a method for inhibiting the propagation of an undesired cell population, the method comprising
(i) introducing an antagonist of SGT into at least one cell of said cell population, ,
   and
(ii) cultivating said cell population for a time period sufficient to allow said SGT antagonist to be effective, thereby inactivating and/or depleting SGT in said cell population
   Optionally, the method of the present invention comprises an additional step, which can be:
(iii) monitoring the growth and/or cell properties of said cell population,
wherein a reduced growth and/or altered cell properties of said cell population as compared to the control cell population is indicative that the propagation of the undesired cell population has been successfully inhibited.

Optionally, the method of the present invention, in particular step (ii), can be performed such that a control cell population which has not being contacted with the SGT antagonist is cultivated in parallel, allowing for a comparison of growth and/or cell properties between the cell population which has been contacted and the cell population which has not been contacted with the SGT antagonist.

Preferably, the method of the present invention is performed in vitro.

Monitoring growth and/or cell properties can occur by several methods known to the person skilled in the art. It includes determining the number of living and/or dead cells within the population, determining the adherence of the cells to the culture dish, determining the shape of the cell, with a detachment and round-up indicating cell death and determining the cell cycle stage of the cell population.

In the context of the present invention, the term "undesired cell population" includes, among others, cell populations which are abnormally dividing, e.g. tumor/cancer cells, cells infected with microorganisms such as bacteria, viruses or yeasts, cells which are affected by toxic substances and autoreactive cells of the immune system. Preferably, the term "an undesired cell population" refers to tumor/cancer cells.

In a preferred embodiment, the cell population which is subjected to the method of the present invention is in the mitotic stage. The inventors have found that SGT particularly acts during mitosis and a depletion of SGT predominantly prevents cells from properly undergoing mitosis.

In a further embodiment of the present invention, the cell population which is subjected to the method of the present invention is in the resting stage (so-called G₀ phase). The inventors have found that the depletion of SGT in cells which are in a resting stage causes intolerance of such cells towards elevated temperatures, i.e. SGT depleted resting cells subjected to higher temperatures die. In a preferred embodiment of the present invention, the elevated temperature ranges between 40 and 45°C, more preferred the elevated temperature is 42°C.

In the context of the present invention, "SGT" refers to all homologs (orthologs and paralogs) the depletion of which results in the phenotype of the present invention (see EXAMPLE section) in the cell population of the respective organism. Included are, e.g., SGT from rat, mouse, yeast, nematode, fruit fly and human (hSGT).

In a preferred embodiment of the present invention the cell population is a cell population of mammalians, such as humans, non-human primates, dogs, cats, cattle, horses, sheep, and the like.

More preferably, the method of the present invention is performed by the depletion of human SGT (hSGT). Consequently, the undesired mammalian cell population is preferably a population of human cells.

The hSGT, as used herein, comprises both a polypeptide as disclosed, e.g., by the NCBI (National Center for Biotechnology Information) accession number CAA11565 (Swiss Prot data base 043765, for hSGT, see SEQ ID NO:1) as well as the nucleic acid encoding it (NCBI number AJ223828, SEQ ID NO:2. Depending on whether SGT refers to a polypeptide or to its encoding nucleic acid, the modes of antagonizing, i.e. depleting SGT, can greatly differ, which will be further described hereinafter.

The term "functional variant" of the SGT polypeptide includes peptides in which one or more amino acids of the original SGT sequence can be substituted by one or more amino acids different from the original one(s), or peptides the amino acid sequence of which is extended or shortened on the aminoterminal and/or the carboxyterminal end or internally, and which still show the proposed effect of inhibiting the propagation of an undesired cell population. Preferably, a functional variant differs from the original amino acid sequence on no more that 50%, more preferably not more than 35% and most preferably not more than 10%.

The term "functional variant" of the SGT nucleic acid includes nucleic acids in which one or more nucleotides of the original SGT DNA sequence can be substituted by one or more nucleotides different from the original one(s), or nucleic acids the nucleotide sequence of which is extended or shortened on the 5'-terminal and/or the 3'-terminal end or internally, and which still show the proposed effect of inhibiting the propagation of an undesired cell population. Preferably, a functional variant differs from the original nucleotide sequence on no more that 50%, more preferably not more than 35% and most preferably not more than 10%.

The term "antagonist" refers to any compound that is capable of directly depleting the expression and/or the function of SGT, either on the nucleic acid level or on the polypeptide level. It is further contemplated within the scope of the present invention that the antagonist refers to any compound that depletes the expression and/or the function of SGT indirectly, e.g. by depleting compounds which are required for proper SGT expression and function.

If the depletion occurs on the nucleic acid level, the antagonist according to the present invention can be a peptide or a nucleic acid that regulates the transcription of the SGT gene by binding to up-stream and/or down-stream regulatory sequences of the coding region of SGT. Such regulatory sequences are known to the person skilled in the art and include so-called promoter, operator, enhancer or silencer regions. For example, the SGT antagonist may interfere with the binding of the RNA polymerase to the promoter region of the SGT gene, either by binding directly to the RNA polymerase binding region, by binding to the polymerase itself or by binding to other factors, e.g. transcription factors, which are required for efficient RNA polymerase binding and function. Furthermore, the SGT antagonist may bind to the operator region and act as a so-called repressor of SGT gene expression.

In a further embodiment of the present invention, the depletion on the nucleic acid level can occur by the use of nucleic acid molecules that hybridize to, and are therefore complementary to the coding sequence of SGT. These nucleic acid molecules may encode or act as SGT gene antisense molecules useful, for example, in SGT gene regulation. With respect to SGT gene regulation, such techniques can be used to modulate, for example, the phenotype and metastatic potential of cancer cells. The use of antisense molecules as inhibitors is a specific, genetically based therapeutic approach. The present invention provides the therapeutic and prophylactic use of nucleic acids of at least six nucleotides that are antisense to a gene or cDNA encoding SGT.

A promising tool to deplete or down-regulate and interfere with the expression of proteins has been developed according to the observation that double-stranded RNA molecules (dsRNA) with homology to a defined sequence within a gene acts as a "trigger" for post-transcriptional gene silencing (PTGS). The unique aspect of this process named "RNA interference" (RNAi) is its exquisite sequence-specificity, leading to the degradation of the targeted mRNA. RNAi has been originally discovered as a mechanism of PTGS in plants and animals. In mammalian cells, RNAi can be achieved by transfecting 19-21 nucleotide (nt) small interfering siRNAs which are complementary to the mRNA sequence of a given target gene, indicating that RNAi may serve as a powerful technology to specifically block the expression of target genes. As such, the use of siRNAs to inhibit the expression of defined target genes has obvious therapeutic potential

Thus, in a preferred embodiment of the present invention, the SGT antagonist of the present invention that depletes the expression and/or the function of a SGT on the nucleic acid level is a dsRNA molecule which is complementary to the SGT mRNA. Preferably, the dsRNA molecules which are complementary to the mRNA of SGT of the present invention have a length between 10 and 30 base pairs, more preferably, they have a length between 19 and 25 base pairs. Most preferred, the siRNA is 21 base pairs in length and corresponds to the sequence as set out in SEQ ID NOs: 3 and 4. The SGT antagonist being siRNA may be delivered to the target cell by any method known the one of skilled art. Applicable is, for instance, the delivery by using cationic liposome reagents.

As the effect of siRNAs, i.e. the reduction of the expression of a certain gene, is considered to be only transient when they are directly applied to cells as described supra it can be advantageous if the nucleic acid, preferably a DNA, encoding the respective SGT siRNA is integrated in an expression vector. Providing suitable elements, as described hereinafter, the DNA is transcribed into the corresponding RNA which is capable of forming the desired siRNA.

The expression vector is preferably a eukaryotic expression vector, or a retroviral vector, a plasmid, bacteriophage, or any other vector typically used in the biotechnology field. If necessary or desired, the nucleic acid can be operatively linked to regulatory elements which direct the synthesis of a mRNA in pro- or eukaryotic cells. Such regulatory elements are promoters, enhancers or transcription termination signals, but can also comprise introns or similar elements, for example those, which promote or contribute to the stability and the amplification of the vector, the selection for successful delivery and/or the integration into the host's genome, like regions that promote homologous recombination at a desired site in the genome. For therapeutic purposes, the use of retroviral vectors has been proven to be most appropriate to deliver a desired nucleic acid into a target cell.

The nucleic acid, preferably a DNA, which is suitable for the preparation of a SGT siRNA can also be introduced into a vector which allows for the production of a double-stranded (ds) RNA molecule. Such vectors are known to the person skilled in the art. To drive the expression of siRNAs these vectors usually contain RNA polymerase III promoters, such as the H1 or U6 promoter, since RNA polymerase III expresses relatively large amounts of small RNAs in mammalian cells and terminates transcription upon incorporating a string of 3-6 uridines. Type III promoters lie completely upstream of the sequence being transcribed which eliminates any need to include promoter sequence in the siRNA. If the DNA encoding the desired siRNA should be transcribed from one promoter, the preferred DNA should thus contain the desired coding region of the SGT gene to be targeted as well as its complementary strand, wherein the coding and its complementary strand are separated by a nucleotide linker, allowing for the resulting transcript to fold back on itself to form a so-called stem-loop structure.

An example for such an expression vector which allows for the production of dsRNA directly in the target cell is the so-called pSUPER (Supression of Endogenous RNA). The vector itself and the mechanism how the dsRNA is produced by using said vector is described in Brummelkamp et al., 2002, Science, Vol. 296, pages 550-553. Another example of such a vector named p*Silencer* (Ambion) was developed by Sui et al., 2002, Proc. Natl. Acad. Sci. Vol. 99, pages 5515-5520.

Furthermore, the present invention encompasses so-called ribozymes as SGT antagonists. Ribozymes are naturally occurring RNA fragments that can be designed as human therapeutics to recognize, bind and digest any disease-causing mRNA sequence, in this case the SGT mRNA. Ribozymes are designed to target the SGT mRNA through complementary base pair hybridization. After binding to the target, the enzymatic activity of the ribozyme cleaves the SGT mRNA thus preventing its translation into protein. The SGT mRNA ribozymes can be chemically synthesized to selectively inhibit SGT production. In addition, the ribozymes may be chemically modified allowing the ribozymes to be more stable and active. Included are also ribozymes that not only cleave SGT-specific RNA molecules but also form carbon-carbon bonds in a covalent fashion, which raises the possibility of ribozymes that can catalyze other types of chemical reactions.

In a further embodiment of the present invention the translation of the SGT gene can be reduced or eliminated by binding of an RNA-binding protein to one or more operator sequences in the 5'-UTR of the SGT mRNA transcript. The bound RNA-binding protein interferes with translation, likely by preventing ribosome assembly or blocking the movement of the ribosome along the transcript from 5' to 3'. Such RNA-binding proteins may be multimeric, e.g. dimers of a particular RNA-binding protein. It is also possible within the scope of the present invention that the SGT antagonist inhibits the SGT expression by promoting or at least being involved in the degradation of SGT mRNA.

If the depletion occurs on the protein level, the present invention encompasses antibodies or fragments thereof capable of specifically recognizing one or more epitopes of the SGT gene products, epitopes of conserved variants of the SGT gene products, epitopes of mutant SGT gene products, or peptide fragments of SGT gene products. Such antibodies may include, but are not limited to, polyclonal antibodies, monoclonal antibodies (mAbs), humanized or chimeric antibodies, single-chain antibodies, Fab fragments, F(ab')2 fragments, Fv fragments, fragments produced by a Fab expression library, anti-idiotypic (anti-Id) antibodies, and epitope-binding fragments of any of the above. The SGT antagonist being an antibody as described above can be used to capture and neutralize SGT in drug-resistant cancer cells. It may be desirable for the present invention if the antibody simultaneously recognizes and neutralizes other proteins than SGT. In order to capture and neutralize more proteins, the antibody used as a SGT antagonist can possess more than one specificities, i.e. being, for example, bispecific, trispecific or multispecific.

Epitopes and antigenic regions useful for generating antibodies can be found within the SGT amino acid sequences (e.g. SWISS PROT data base number 043765; SEQ ID NO:1) by procedures available to one of skill in the art. For example, short, unique peptide sequences can be identified in the amino acid sequences that have little or no homology to known amino acid sequences. Preferably the region of a protein selected to act as a peptide epitope or antigen is not entirely hydrophobic; hydrophilic regions are preferred because those regions likely constitute surface epitopes rather than internal regions of the present proteins and polypeptides. These surface epitopes are more readily detected in samples tested for the presence of the present proteins and polypeptides.

Peptides can be made by any procedure known to one of skill in the art, for example, by using in vitro translation or chemical synthesis procedures. Short peptides which provide an antigenic epitope but which by themselves are too small to induce an immune response may be conjugated to a suitable carrier. Suitable carriers and methods of linkage are well known in the art. Suitable carriers are typically large macromolecules such as proteins, polysaccharides and polymeric amino acids. Examples include serum albumins, keyhole limpet hemocyanin, ovalbumin, polylysine and the like. One of skill in the art can use available procedures and coupling reagents to link the desired peptide epitope to such a carrier. For example, coupling reagents can be used to form disulfide linkages or thioether linkages from the carrier to the peptide of interest. If the peptide lacks a disulfide group, one may be provided by the addition of a cysteine residue. Alternatively, coupling may be accomplished by activation of carboxyl groups.

The minimum size of peptides useful for obtaining antigen specific antibodies can vary widely. The minimum size must be sufficient to provide an antigenic epitope which is specific to the protein or polypeptide. The maximum size is not critical unless it is desired to obtain antibodies to one particular epitope. For example, a large polypeptide may comprise multiple epitopes, one epitope being particularly useful and a second epitope being immunodominant.

In a further embodiment of the present invention, the SGT antagonist that depletes the expression and / or the function of the SGT can be a so-called aptamer, either a peptide-based aptamer or an oligonucleotide-based aptamer. Peptide aptamers are defined as protein-based recognition agents that consist of constrained combinatorial peptide libraries displayed on the surface of a scaffold protein (e.g. thioredoxin). Peptide aptamers function *in trans,* interacting with and inactivating gene products without mutating the DNA that encodes them. In principle, combinatorial libraries of peptide aptamers should contain aptamers that interact with any given gene product, thus allowing peptide aptamers to be generated against an organism's entire proteome. Oligonucleotide-based aptamers being used as SGT antagonist according to the present invention comprise DNA as well as RNA aptamers. In this respect, the present invention encompasses also mirror-image L-DNA or L-RNA aptamers, so-called spiegelmers. The aptamers that are also useful as SGT antagonists for the present invention include those which interact with specific proteins and thus prevent or disrupt the specific protein interaction between the SGT and its possible interaction partner.

In the context of the described aptamers, it is also feasible that the SGT antagonist comprises so-called small molecule inhibitors that may exhibit similar properties as aptamers, namely binding to either the SGT or to an interacting partner, thereby inhibiting their proper interaction and, thus, function. The small molecule inhibitor can be a peptide or a small chemical compound, which has been identified by methods known to the skilled artisan, e.g. by computational combinatorial chemistry in combination with screening of compound libraries. Furthermore, the depletion of SGT can be achieved by using so-called muteins of SGT. Muteins are derivatives of biologically active proteins the amino acid composition of which has been artificially altered. The muteins can be made via bacterial expression of mutant genes that encode the muteins that have been synthesized from the genes for the parent proteins by oligonucleotide-directed mutagenesis.

The person skilled in the art is aware of a variety of methods how to introduce the disclosed SGT antagonists into the target cell. In general, the appropriate method depends on whether the SGT antagonist is a nucleic acid or a peptide. Furthermore, if the SGT antagonist is a peptide it can be delivered into the target cell by introducing the nucleic acid encoding it.

There are several well-known methods of introducing nucleic acids into animal cells, any of which may be used in the present invention and which depend on the host. Typical hosts include mammalian species, such as humans, non-human primates, dogs, cats, cattle, horses, sheep, and the like. At the simplest, the nucleic acid can be directly injected into the target cell / target tissue, or by so-called microinjection into the nucleus. Other methods include fusion of the recipient cell with bacterial protoplasts containing the nucleic acid, the use of compositions like calcium chloride, rubidium chloride, lithium chloride, calcium phosphate, DEAE dextran, cationic lipids or liposomes or methods like receptor-mediated endocytosis, biolistic particle bombardment ("gene gun" method), infection with viral vectors, electroporation, and the like.

For the introduction of the SGT antagonist, respectively the nucleic acid encoding it, into the cell and its expression it can be advantageous if the nucleic acid is integrated in an expression vector. The expression vector is preferably a eukaryotic expression vector, or a retroviral vector, a plasmid, bacteriophage, or any other vector typically used in the biotechnology field. If necessary or desired, the nucleic acid encoding the SGT antagonist can be operatively linked to regulatory elements which direct the transcription and the synthesis of a translatable mRNA in pro- or eukaryotic cells. Such regulatory elements are promoters, enhancers or transcription termination signals, but can also comprise introns or similar elements, for example those, which promote or contribute to the stability and the amplification of the vector, the selection for successful delivery and/or the integration into the host's genome, like regions that promote homologous recombination at a desired site in the genome. For therapeutic purposes, the use of retroviral vectors has been proven to be most appropriate to deliver a desired nucleic acid into a target cell. Preferably, the vectors contain tumor- or tissue specific promoters, or promoters which are specifically activated in the presence of a pathogen.

Particularly advantageous for the present invention is the introduction of the SGT antagonist, particularly the nucleic acids encoding the same, by the use of tumor-specific viruses or engineered viruses the specific targeting of cells of cells (oncotropic viruses, like e.g. parvovirus, viruses which are conjugated to albumin, modified adenovirus, New Castle Disease virus, reovirus, measles virus, recombinant Herpes virus). Other suitable delivery systems include those which allow the specific targeting of tumor cells, e.g. albumin-coated liposomes

The introduction of a nucleic acid encoding an SGT antagonist can be facilitated by the use of so-called translocating peptides. The use of such peptides is particularly preferred for therapeutic purposes. The peptides are usually linked to the nucleic acid to be delivered in a non-covalent manner. In general, peptides are contemplated which mimic and act as efficiently as viruses for gene delivery without their limitations of inducing immune responses or being cytotoxic. Examples of peptides forming peptide-DNA complexes for an efficient delivery into a cell comprise DNA-condensing motifs such as polyamines and modifications thereof, active-targeting motifs such as RGD, endosomolytic peptides such as INF, JTS1 or GALA, and nuclear localization sequences (NLS), e.g. derived from the large tumor antigen of Simian 40 virus. An extensive list of translocating peptides and their proposed delivery mechanisms, all of which are contemplated within the scope of the present invention are described in Morris et al., 2000, Curr. Opin. Biotech., Vol. 8, pages 21 to 27.

If the SGT antagonist is a peptide that shall be directly introduced into the target cell it can be fused to a carrier peptide that mediates the cellular uptake of the peptide. Appropriate carriers are known to the person skilled in the art and include TAT, fibroblast growth factor, galparan (transportan), poly-arginine, and Pep-1. Furthermore, SGT antagonist may be fused to a ligand for a cell surface receptor, or a functional portion thereof, and thus internalized by receptor-mediated endocytosis.

The time period sufficient to allow the SGT antagonist to be effective depends on the nature of the candidate compound, i.e. whether it is a small molecule inhibitor, a nucleic acid, a peptide, a protein or other, as specified in detail supra and has to be determined experimentally. If the SGT antagonist is a siRNA it is preferred that the cell population is cultivated at least 20 hours, more preferably at least 40 hours, most preferred at least 70 hours in order allow the SGT antagonist to be effective.

The SGT antagonist, which is employed for the method of the present invention, namely the depletion of an undesired cell population, can be used for the manufacture of a medicament for the treatment of a disease which is caused by the propagation of an undesired cell population. In a preferred embodiment of the present invention, the disease which is caused by the propagation of an undesired cell population is cancer.

Examples of cancers comprise neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, follicular thyroid carcinoma, anaplastic thyroid carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma. ,

More preferably, the cancer to be treated with a medicament employing a SGT antagonist is selected from the group consisting of cervical carcinoma, neuroblastoma, glioblastoma and breast carcinoma.

In a further embodiment of the present invention, the SGT antagonist, which is employed for the method of the present invention, namely the depletion of an undesired cell population, for example autoreactive cells of the immune system, can be used for the manufacture of a medicament for the treatment of autoimmune diseases.

Examples of such autoimmune diseases are collagen diseases such as rheumatoid arthritis, Lupus erythematodes disseminatus, Sharp syndrome, CREST syndrome (calcinosis, Raynaud syndrome, esophageal dysmotility, teleangiectasia), dermatomyositis, vasculitis (Morbus Wegener) and Sjögren syndrome, renal diseases such as Goodpasture syndrome, rapidly-progressing glomerulonephritis and membrane-proliferative glomerulonephritis type II, endocrine diseases such as type-I diabetes, autoimmune polyendocrinopathy-candidiasis-ectodermal dystrophy (APECED), autoimmune parathyreoidism, pernicious anemia, gonad insufficiency, idiopathic Morbus Addison, hyperthyreosis, Hashimoto thyreoiditis and primary myxedemia, skin diseases such as Pemphigus vulgaris, bullous pemphigoid, Herpes gestationis, Epidermolysis bullosa and Erythema multiforme major, liver diseases such as primary biliary cirrhosis, autoimmune cholangitis, autoimmune hepatitis type-I, autoimmune hepatitis type-2, primary sclerosing cholangitis, neuronal diseases such as multiple sclerosis, Myastenia gravis, myasthenic Lambert-Eaton syndrome, acquired neuromyotony, Guillain-Barré syndrome (Müller-Fischer syndrome), Stiff-man syndrome, cerebellar degeneration, ataxia, opsoklonus, sensoric neuropathy and achalasia, blood diseases such as autoimmune hemolytic anemia, idiopathic thrombocytopenic purpura (Morbus Werlhof), infectious diseases with associated autoimmune reactions such as Malaria and Chagas disease.

The medicaments according to the invention can be administered orally, for example in the form of pills, tablets, lacquered tablets, sugar-coated tablets, granules, hard and soft gelatin capsules, aqueous, alcoholic or oily solutions, syrups, emulsions or suspensions, or rectally, for example in the form of suppositories. Administration can also be carried out parenterally, for example subcutaneously, intramuscularly or intravenously in the form of solutions for injection or infusion. Other suitable administration forms are, for example, percutaneous or topical administration, for example in the form of ointments, tinctures, sprays or transdermal therapeutic systems, or the inhalative administration in the form of nasal sprays or aerosol mixtures, or, for example, microcapsules, implants or rods. The preferred administration form depends, for example, on the disease to be treated and on its severity.

The preparation of the medicaments can be carried out in a manner known per se. To this end, the SGT antagonist, together with one or more solid or liquid pharmaceutical carrier substances and/or additives (or auxiliary substances) and, if desired, in combination with other pharmaceutically active compounds having therapeutic or prophylactic action, are brought into a suitable administration form or dosage form which can then be used as a pharmaceutical in human or veterinary medicine.

For the production of pills, tablets, sugar-coated tablets and hard gelatin capsules it is possible to use, for example, lactose, starch, for example maize starch, or starch derivatives, talc, stearic acid or its salts, etc. Carriers for soft gelatin capsules and suppositories are, for example, fats, waxes, semisolid and liquid polyols, natural or hardened oils, etc. Suitable carriers for the preparation of solutions, for example of solutions for injection, or of emulsions or syrups are, for example, water, physiological sodium chloride solution, alcohols such as ethanol, glycerol, polyols, sucrose, invert sugar, glucose, mannitol, vegetable oils, etc. It is also possible to lyophilize the SGT antagonist and to use the resulting lyophilisates, for example, for preparing preparations for injection or infusion. Suitable carriers for microcapsules, implants or rods are, for example, copolymers of glycolic acid and lactic acid.

The pharmaceutical preparations can also contain additives, for example fillers, disintegrants, binders, lubricants, wetting agents, stabilizers, emulsifiers, dispersants, preservatives, sweeteners, colorants, flavorings, aromatizers, thickeners, diluents, buffer substances, solvents, solubilizers, agents for achieving a depot effect, salts for altering the osmotic pressure, coating agents or antioxidants.

The dosage of the SGT antagonist to be administered, depends on the individual case and is, as is customary, to be adapted to the individual circumstances to achieve an optimum effect. Thus, it depends on the nature and the severity of the disorder to be treated, and also on the sex, age, weight and individual responsiveness of the human or animal to be treated, on the efficacy and duration of action of the compounds used, on whether the therapy is acute or chronic or prophylactic, or on whether other active compounds are administered in addition to the SGT antagonist.

A further embodiment of the present invention refers to a method for screening candidate compounds for at least one SGT antagonist with the ability to inhibit the propagation of a cell population, the method comprising the following steps:
(i) contacting a cell population with a candidate compound, thereby enabling the introduction of said candidate compound into the cells of said cell population,
(ii) cultivating said cell population for a time period sufficient to allow the candidate compound to be effective, and, in parallel, cultivating a control cell population which has not been contacted with the candidate compound,
   and
(iii) monitoring cell growth and/or cell properties in said cell population and in the control cell population,
wherein a reduced growth and/or altered cell properties as compared to the control cell population is indicative that the candidate compound is a SGT antagonist which inhibits the propagation of a cell population.

In a preferred embodiment, the method comprises the additional steps:
(iv) qualitatively and/or quantitatively detecting the SGT expression in said cell population and in the control cell population, wherein a lower level of SGT expression is indicative of a compound that is a SGT antagonist,
   and
(v) determining whether a lower level of SGT expression correlates with a reduced growth and/or altered cell properties of the cell population being contacted with the candidate compound.

The time period sufficient to allow the candidate compound to be effective depends on the nature of the candidate compound, i.e. whether it is a so-called small molecule inhibitor, a nucleic acid, a peptide, a protein or other, and has to be determined experimentally. Ideally, the time period may last at least one cell division cycle in order to verify an effect of the candidate compound on cell growth.

The detecting of the SGT expression can be performed by methods known to the skilled artisan. If the SGT expression is detected on the protein level, methods like Western Blotting, ELISA, RIA, metabolic labelling and immunoprecipitation employing suitable SGT-specific antibodies can be performed. If the SGT expression is detected on the nucleic acid level, hybridization techniques, like Southern or Northern Blotting, employing suitable SGT-specific probes can be performed. For the detection of SGT expression, it is useful if the detecting molecules, e.g. nucleic acid probes or antibodies, are labelled. Suitable labelling methods/agents include radioactive labelling, fluorescent labelling, attachment of an enzyme moiety the activity of which is measured, attachment of tags (e.g. myc, FLAG, His, biotin).

The present invention further refers to a method for the preparation of a pharmaceutical composition wherein a SGT antagonist inhibiting the propagation of an undesired cell population is identified according to the screening method as specified above, synthesized in adequate amounts, and formulated into a pharmaceutical composition.

In this respect, the present invention also encompasses a pharmaceutical composition manufactured by identifying a SGT antagonist according to the screening method as specified above, synthesizing it in adequate amounts, and formulating it into a pharmaceutical composition.

The present invention further refers to the use of a SGT antagonist for inhibiting the propagation of an undesired cell population, which is, preferably, in the mitotic stage. In a preferred embodiment, the cell population is a cell population of human cells.

### DESCRIPTION OF THE DRAWING

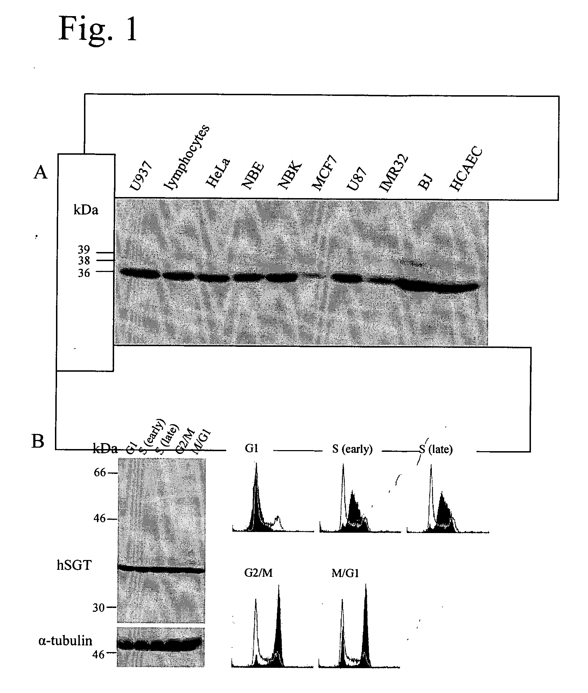

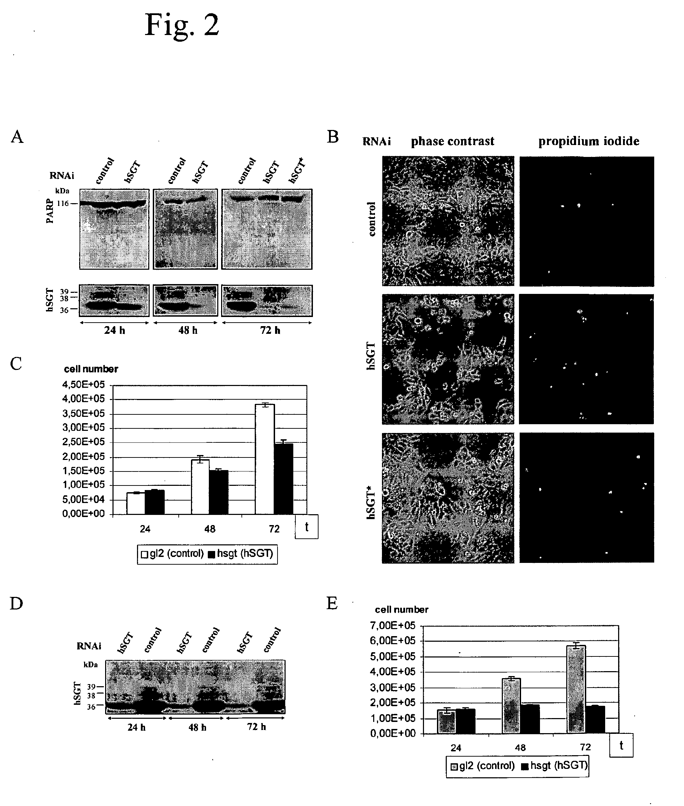

Figure 1: hSGT protein was present in various human cell lines and throughout the cell cycle.
   (A) A panel of different human culture cell lines (immortalized or transformed) was assessed for the presence of hSGT protein by Western blotting using hSGT-specific antibodies after fractionation of total extracts from equal number of cells in SDS-PAGE.
   (B) HeLa cells were synchronized through thymidine (2 mM) treatment. Total extracts from equal number of cells were analyzed at different time points (0 h, 2 h, 4 h, 5 h and 7.5 h) after release from the double thymidine block. The corresponding cell cycle phases were determined through FACS analysis
Figure 2: Proliferation of hSGT-depleted cell populations was reduced.
   (A) Reduction of hSGT protein levels after siRNA treatment. NBE cells were transfected with *gl2-* (control lanes) or either of two *hsgt*-specific siRNAs (hSGT and hSGT* lanes) and harvested 24 h, 48 h and 72 h after transfection. Extracts from equal amounts of cells were subjected to protein immunoblotting, using either a hSGT-specific antiserum or - in order to control for gel loading and occurrence of apoptosis - a PARP specific antibody.
   (B) Impaired growth of hSGT knock down cultures. NBE cells were analyzed by phase contrast and epifluorescence (after propidium iodide staining) microscopy, 72 h after transfection with *gl2*- (control) or *hsgt*-specific (hSGT or hSGT*) siRNAs. Compared to controls, the following effects were observed in hSGT-depleted populations: detachment of cells, increase in dead cells (propidium iodide stained) and lower cell density. (C) Quantification of NBE cells in populations transfected either with *gl2-* (control) or *hsgt*-(hSGT) siRNA 24 h, 48 h, and 72 h after transfection. (D) Reduction of hSGT protein levels after siRNA transfection in HeLa cells. (E) Quantification of HeLa cells in populations transfected either with *gl2-* (control) or *hsgt-* (hSGT) siRNA 24 h, 48 h, and 72 h after transfection.
Figure 3: hSGT-depleted cell population showed a complex cell cycle distribution with a decrease in the G1- and an increase in the G2/M-fraction size.
   (A) Time course of changes in cellular DNA content after *hsgt*-specific siRNA treatment. For comparison, untreated and control (*gl2*) transfected cells were analyzed. (B) Quantification of cell cycle fractions in percent.
Figure 4: hSGT knock down populations showed an increased mitotic index, yet lacked continued accumulation in M-phase, due to cell death occurring at later time points after transfection.
   (A) Determination of mitotic indices in NBE cell populations that were either untreated or transfected with *hsgt-* (hSGT) or *gl2-* (control) specific siRNA. Cultures were examined by phase contrast and epifluorescence (Hoechst 33342 staining) microscopy, and images were obtained at the indicated time points after transfection. Box: Rounded cell with condensed chromatin (Hoechst 33342) and smooth uniform membranes, as well as cells in ana- or telophase were scored as mitotic. Rounded and detached cells without clearly visible chromosomes and without intact membranes were rated as dead. (B) Quantification of mitotic and dead cells in the non-adherent fraction of hSGT-depleted cell populations 48 h and 72 h after transfection.

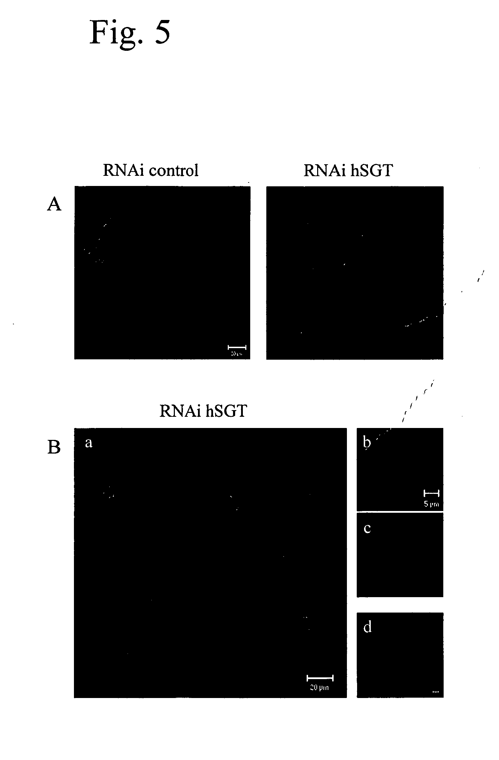

Figure 5: A significant proportion of cells in the non-adherent fraction of hSGT-depleted populations contained mitotic structures, while an increased proportion of binucleated cells were detected in the adherent fraction.
   (A) Adherently growing NBE cell populations were fixed at 72 h after transfection with either *gl2-* or *hsgt-* specific siRNA. Samples were stained with α-tubulin-specific antibodies, and analyzed by confocal microscopy (bar = 20 µm). (B) Cells from the non-adherent fraction of *hsgt-* (a-c) or *gl2-* (control, d) siRNA transfected populations were collected and transferred to coverslips as described in Materials and Methods. For confocal analysis, cells were fixed and stained with antibodies to α-tubulin (a-d), while Hoechst 33342 was used to visualize the DNA (a). Representative overviews of supernatants from *hsgt-* (a) or *gl2-* (d) specific siRNA-treated cell populations (bar = 20 µm). Typical mitotic structures in non-adherent hSGT knock down cells (b and c, bar = 5 µm)

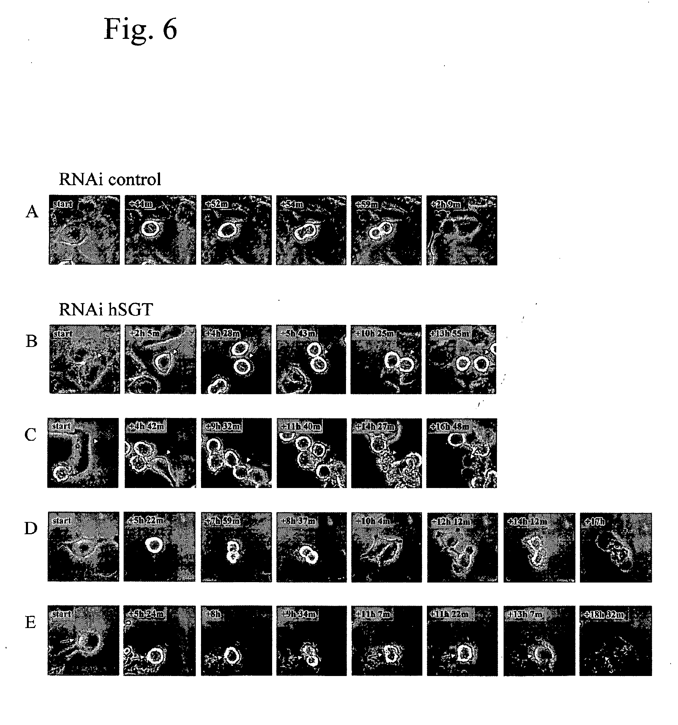

Figure 6: NBE cells from hSGT-depleted populations showed defects in the completion of cell division.
   Progress through mitosis of *gl2* (control) siRNA (A) versus *hsgt* siRNA (B-E) transfected cells. Cell populations were analyzed in a time-period between 30 h and 90 h after transfection, using time-lapse video microscopy. Typical examples of cell fates obtained in hSGT knock down cell populations are depicted (B-E). Compared to controls (A), affected cells consistently displayed arrest in prometa- or metaphase (B), arrest in mitosis followed by cell death (C), death in early G1, before midbody abscission (D), incomplete cytokinesis generating a binucleated cell (E). Time is given in hours (h) and minutes (m)

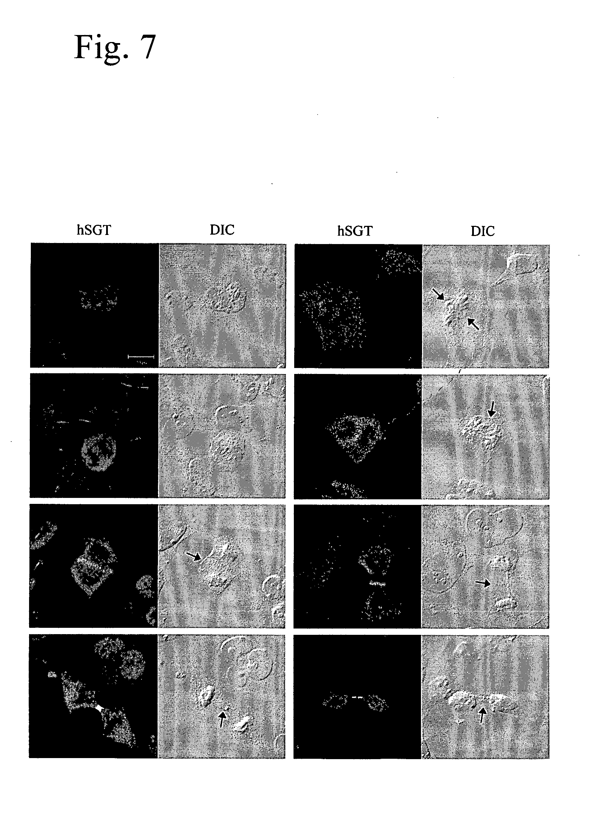

Figure 7: Distribution of hSGT protein in the course of mitosis.
   NBK cells were stained with hSGT-specific antibodies, while DIC was used to monitor the status of the chromatin. The images were obtained by confocal microscopy. hSGT showed no site-specific enrichment in prophase (A) until the onset of anaphase (C), except for a weak accumulation at the spindle poles in metaphase (B, arrows). During ana- (D and E) and early telophase (F), hSGT was localized between the chromosomes (D, E and F, arrows), while it concentrated in the midbody until cytokinesis was completed (G and H, arrows). Bar = 10 µm.

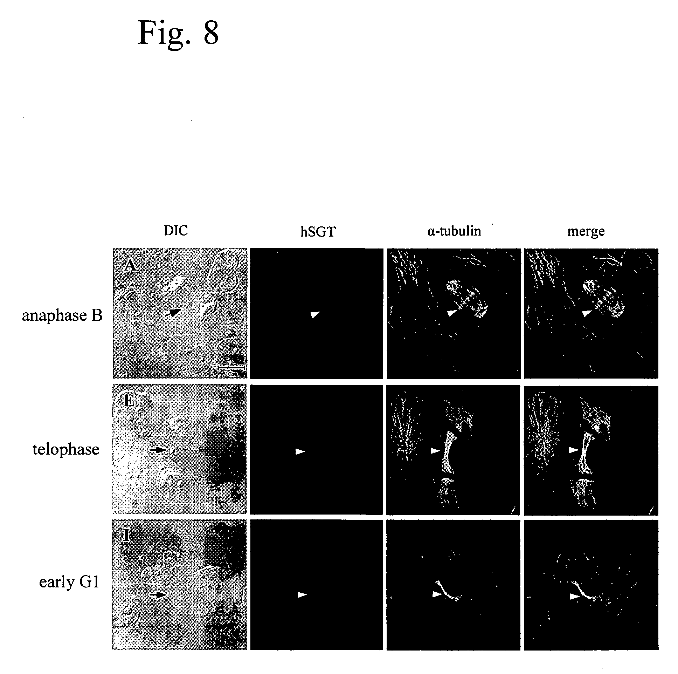

Figure 8: hSGT accumulated in the midzone and the midbody.
   NBK cells were fixed with 2% formaldehyde and incubated with hSGT- (red; B, F, and J) and α-tubulin- (green; C, G, and K) specific antibodies. DIC was used to visualize the chromatin (A, E and I). Images were obtained by confocal microscopy. Merged images highlight the areas of colocalisation (D, H, and L). During anaphase, hSGT clearly accumulated in the midzone (D). In late telophase (H) and in early G1 (L), hSGT- and α-tubulin-specific signals were detected in the midbody. Bar = 10 µm.

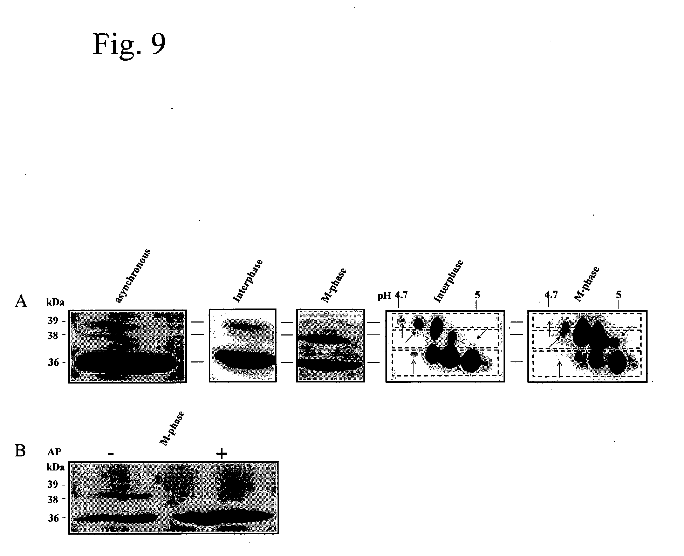

Figure 9: hSGT displayed a mitosis-specific migration pattern.
   Extracts from equal amount of cells were fractionated and subjected to protein immunoblot analysis with hSGT specific antibodies. (A) Comparison of hSGT polypeptides from asynchronous, interphase or mitotic NBK cell populations after separation through SDS-PAGE with or without prior isoelectric focusing. (B) Comparison of hSGT polypeptides treated or not with alkaline phosphatase prior to SDS-PAGE

The invention is further illustrated by the following examples:

### EXAMPLES

### Example 1: Cell culture

The human culture cell lines HeLa (cervical-carcinoma), NBE, NBK (embryonic kidney epithelial cells, SV40 transformed), U87 (glioblastoma), BJ (primary foreskin fibroblasts), IRM32 (neuroblastoma with N*myc* amplification), MCF7 (mammary carcinome cells), U937 (promyeolocytic cells), immortalized lymphocytes (Epstein-Barr-Virus transformed) were obtained from ATTC and cultured as described while HCAEC (primary coronary artery endothelial cells) were obtained from PromoCell (Heidelberg, Germany) and cultured as recommended by the supplier. For microscopic analyses, NBE and NBK cells were used. Epithelial features of NBE cells were confirmed based on keratin- and vimentin-specific staining patterns, while the desmoplakin-specific staining reflected the embryonic character of these cells. Due to their superior transfection rate as compared to NBK, NBE (and also HeLa) cells were chosen for RNAi experiments. For the detection of hSGT throughout the cell cycle, synchronized HeLa cells were obtained by double thymidine treatment (2 mM). Prior to harvesting, cells were released from the second thymidine block after 0 h, 2 h, 3 h, 5 h and 7.5 h. For growth curves or in order to normalize gel-loading, cells were counted in Neubauer chambers.

### Example 2: Immunological reagents

hSGT-specific serum, AC1.1 and AG1.2, were generated and affinity-purified essentially as described. In brief, rabbit serum was raised against purified, bacterially expressed His-tagged rat SGT (AC1.1) or human SGT (AG1.2). Antibodies were affinity-purified by binding IgGs to CNBr-sepharose coupled His-tagged rat SGT (AC1.1) or human SGT (AG1.2), eluted with 0.2 M glycine-NaOH (pH 2.1) and immediately neutralized through the addition of Tris-HCl (pH 8.8). The specificity of the AG1.2 and AC1.1 antibodies was shown through RNAi-experiments: after hSGT knock down, a time-dependent drastic reduction of hSGT-specific signals was observed in Western blotting and immunofluorescence analysis.

### Example 3: RNA interference

Two distinct small interfering RNA (siRNA) oligonucleotides duplexes (hSGT and hSGT*,) which specifically targeted the sequences 5'-AACTTGAAGCTGCCGTGGATT-3' (SEQ ID NO:2) and 5'-AAGCACGTGGAGGCCGTGGCTTA-3' (SEQ ID NO:3) within *hsgt* were supplied by Xeragon Inc.. By BLAST analysis, the sequences of these siRNA oligonucleotides were not detected in any other human gene. As negative control, the previously described *gl2*-specific siRNA was used. Transfection was performed using the oligofectamine™ Reagent (Invitrogen) following the instruction of the supplier, and silencing efficiency was determined by evaluating the residual amount of hSGT after immunoblotting.

### Example 4: Cell lysates and immunoblotting

To obtain protein extracts, asynchronously growing cell populations were harvested after trypsin treatment. Mitotic cells were obtained through mitotic shake-off and the remaining adherent cells yielded the interphase fraction. Cells were counted, collected through centrifugation, washed in PBS and lysed in 2x Laemmli buffer. Samples corresponding to 5x10⁵ cells were fractionated on 10% SDS-polyacrylamide gels and transferred to a nitrocellulose membrane (Schleicher & Schuell). Polypeptides were detected with either the hSGT-specific AG1.2 antiserum (1:500) or the PARP-specific antibody F1/23 (1:5), kindly provided by Dr. A. Bürkle (University of Konstanz, Constance). Detection through enhanced chemiluminescence was performed as recommended by the supplier (Amersham Biosciences), after incubation with a peroxidase-conjugated secondary antibody (1:5000).

For 2-D analysis, 5x10⁶ interphase or mitotic cells were incubated for 30 min at 37°C with lysis buffer containing 9 M urea, 4% CHAPS (3-[(3-Cholamidopropyl)dimethylammonio]-1-propanesulfonate), 10 mM DTT and 0.5% Ampholine (pH 4-7). Cell lysates were passed through QIA-shredder™ following the instructions of the supplier (Qiagen). Samples corresponding to 700 µg protein were applied to Immobiline Drystrips (Amersham Biosciences) and subjected to in-gel rehydration successively for 12 h without an electric field, 1 h at 500 V and 1 h at 1000 V, in an IPGphor electrophoresis chamber (Amersham Biosciences). Isoelectric focusing was performed at 8000 V until 80000 Vh were reached. Strips were equilibrated for 20 min in 50 mM Tris-HCl (pH 8.8), 6 M urea, 30% gycerol, 2% SDS and 0.02% bromophenolblue. Separation in the second dimension was performed in 10% SDS-PAGE.

For alkaline phosphatase treatment, cell extract was prepared from a total of 1x10⁶ cells. After harvesting, cells were washed in PBS, resuspended in a hypotonic buffer (20 mM HEPES-KOH [pH 7.5], 7.5 mM MgCl₂, 0.1 mM DTT), incubated on ice for 20 min and passaged through a tight-fitting Dounce homogenizer. Proteins were extracted in 750 mM NaCl for 30 min on ice, and the extract was subjected to clear spin prior to use. 30-50 µg protein were incubated with 17 IU alkaline phosphatase (Roche) in dephosphorylation buffer containing 100 mM ZnCl₂, 10 mM MgCl₂ and 100 mM Tris-HCl (pH 8.0) for 45 min at 37°C and subsequently fractionated through 10% SDS-PAGE.

### Example 5: Flow cytometry

For FACS analysis, NBE or HeLa cells were grown in culture dishes and depending on the experiments, transfected or not with either control siRNA (*gl2*; Elbashir et al., 2001) or *hsgt*-specific siRNA. In case of transfection, non-adherent and adherent cells were collected and combined at 48 h and 72 h post-transfection. After three washes in PBS, cells were fixed in 80% ice-cold methanol and stored at -20°C. For analysis, cells were pelleted, washed with PBS, resuspended in 500 µl PBS containing 100 µg/ml RNase and incubated for 30 min at 37°C. Then, 500 µl PBS containing 100 µg/ml propidium iodide was added. Cell cycle distribution of the cells was determined by analyzing their DNA content with a Becton Dickinson FACScan®.

### Example 6: Immunofluorescence microscopy

NBK cells were grown on glass coverslips and fixed with PFA solution (PBS buffer containing 2% formaldehyde) for 15 min at room temperature. They were then washed with PBS and permeabilized with 0.2% Triton X-100. After successive washing with PBS-TN buffer (PBS containing 0.2% Tween 20 and 0.2% NP40) and PBS-M buffer (PBS containing 2 mM MgCl₂) twice each, fixed cells were pretreated with PBS containing 1% BSA for 20 min. Cells were then incubated for 1 h with primary antibodies directed against hSGT (AC1.1 [rabbit], 1:5) or α-tubulin (DM1A [mouse], Sigma, 1:1000). Coverslips were successively rinsed three times in PBS-TN, and PBS-M buffer and then incubated for 1 h with secondary antibodies labeled with either Cy5, Alexa®488 or TRITC. Cellular DNA was visualized by staining with Hoechst 33342 (2 µg/ml). Samples were analyzed using a LSM500 confocal microscope (Zeiss).

In the case of RNAi-treated NBE cells, two different fractions (adherent and non-adherent) were analyzed 72 h after transfection. The adherently growing cells were processed directly on the coverslips onto which they had been seeded. In contrast, non-adherent and suspended cells were collected through gentle aspiration, pelleted, resuspended in a reduced volume and transferred to adhesion-slides (BioRad). In order to enhance transfection rates, these cells were treated a second time with siRNA 20 h after the first transfection.

### Example 7: Phase contrast microscopy and time-lapse imaging

To determine the fraction of dead cells or mitotic and binucleated indices, cells were incubated either with 10 µg/ml propidium iodide or 8 µg/ml Hoechst 33342 for 10 min. Images were obtained 48 h and 72 h after transfection by epifluorescence microscopy (Ex 535/30, DM 565, EM 590 LP, for propidium iodide and Ex 340/40, DM 390, EM 400LP for Hoechst 33342 stained cells) and by phase contrast microscopy using a Zeiss 10x phase contrast objective on a Zeiss Axiovert S 100TV microscope. For image acquisition, the openlab software (Improvision Inc.) was used.

For time-lapse video microscopy, cells were cultivated in a 6-well dish and transfected with *gl2*- or hsgt-specific siRNA. At 30 h, 48 h and 72 h post-transfection dishes were placed on the microscope stage and maintained at 37°C using a tempocontrol-37-2 chamber heater and at 5% CO₂ atmosphere. Cells were time-lapse recorded using a Zeiss 10x phase contrast objective on a Zeiss inverted Axiovert S 100TV stand. Images (680 x 680 µm frames) were acquired with a Hamamatsu digital camera at 150 s or 200 s intervals for 16.5 h or 24 h, using the openlab software.

### Example 8: hSGT protein was detected in all human cell lines tested and protein levels appeared to remain constant throughout the cell cycle

Transcripts from SGT-encoding genes were previously detected in various different tissues from rat and human. In order to test whether the hSGT protein was also ubiquitously present, a panel of human cultured cell lines (primary and transformed) was analyzed by Western blotting for the presence of this protein (Fig. 1 A). The panel included the following cell lines: HeLa (cervical carcinoma), NBK and NBE (SV40-transformed new born human kidney epithelial cells), IMR32 (neuroblastoma with N*myc*-amplification), U87 (glioblastoma), BJ (primary fibroblasts), MCF7 (breast cancer), U937 (promyeolocytic cells), "primary" lymphocytes (Epstein-Barr-Virus transformed) and primary HCAEC (human coronary artery endothelial cells). Western blot analysis revealed the presence of hSGT protein in all cell lines tested. As previously reported, three fractions of hSGT protein with apparent molecular weights of 36, 38 and 39 kDa could be distinguished in asynchronously growing cells after SDS-PAGE and Western blotting, the relative signal intensities of which showed some variation between the cell lines analyzed (Fig. 1 A).

In order to determine the presence of hSGT protein throughout the cell cycle, total cell extracts of synchronized HeLa cells from fractions corresponding to G1, early and late S, G2/M and M/G1 phases - as determined by fluorescence-activated cell sorting (FACS) analysis - were generated and subjected to Western blotting (Fig 1. B). These analyses showed that the hSGT protein was detectable throughout the cell cycle, with only a very slight increase in the intensity of the band corresponding to the 38 kDa fraction in cells from the M/G1 population. In summary, the results indicated that hSGT protein is a constitutive component of all human cell lines tested, suggesting a possible role as a housekeeping protein.

### Example 9: hSGT depletion interfered with cell population growth

To identify cellular processes in which hSGT is involved, this protein was depleted in the NBE cell line after transfection with *hsgt*-specific small interfering RNA oligonucleotides (siRNA). The phenotype of hSGT-depleted cell populations was compared to that of control populations expressing regular levels of hSGT (Fig. 2 A). Controls were generated through transfection of NBE cells with a previously described *luciferase*-specific siRNA (*gl2*) proven to have no effect on human cells.

After treatment of NBE cells with *hsgt*-specific siRNA, a significant and strong reduction of hSGT protein levels could be achieved as demonstrated through Western blot analysis (Fig. 2 A). Reduced hSGT protein levels were detected at least up to 72 h after transfection. Already 24 h after transfection, a slight decrease in hSGT levels could be detected, while at 48 h and 72 h a very significant reduction (by at least 80%) was observed compared to controls (Fig. 2 A).

When controls with regular hSGT protein levels and cell populations with experimentally reduced hSGT protein levels were analyzed by phase contrast microscopy, several striking differences could be observed 72 h after transfection (Fig. 2 B). Most prominently, a significant fraction of hSGT-depleted cells did not adhere to the culture dish anymore but displayed a rounded-up and detached phenotype. Staining with propidium iodide revealed that many of these non-adherent cells were dead (Fig. 2 B). Although the precise mode of cell death remains to be determined, the absence of detectable Poly(ADP-ribose) polymerase (PARP) cleavage (Fig. 2 A) - a commonly used marker for caspase-dependent apoptosis - suggested that hSGT depletion-dependent death was not induced via a classical apoptotic pathway in this cell line. Finally, hSGT knock down cell populations (adherent and non-adherent cells) appeared to have grown to a lower cell density in comparison to the control population (Fig. 2 B), although equal amounts of cells had been initially seeded.

The specificity of the observed phenotype of hSGT-depleted cell populations was verified - in analogy to a previous report - by the use of two distinct *hsgt*-specific siRNAs targeting different regions within the transcript. Transfection with either siRNA (hSGT or hSGT*) resulted in a comparable reduction of hSGT protein levels (Fig. 2 A) and most importantly in an identical phenotype, such as detachment of cells, reduced cell density, and increased cell death (Fig. 2 B), thereby clearly demonstrating phenotype specificity.

To quantify the microscopically observed differences in cell number between hSGT-depleted and control populations, all cells (non-adherent and adherent) were counted in both cases. Compared to controls, the total number of cells was reduced in the hSGT-depleted populations by about 20% at 48 h and by 36% at 72 h after transfection (Fig. 2 C). It is worth noting that culture growth was not totally impaired in the hSGT-depleted populations, which could be due to incomplete transfection with hsgt-specific siRNA. Besides the NBE cell line, other human cell lines (e.g. HeLa) were transfected with *hsgt*-specific or control siRNA which resulted in comparable reductions of hSGT protein levels (Fig. 1 D) and also to a strong reduction in the growth of treated cell populations (Fig. 1 E). From these results it was concluded that under the experimental conditions tested, hSGT protein became limiting for the growth of human cell cultures.

### Example 10: hSGT-depleted cell populations showed increases in the fraction of mitotic and binucleated cells

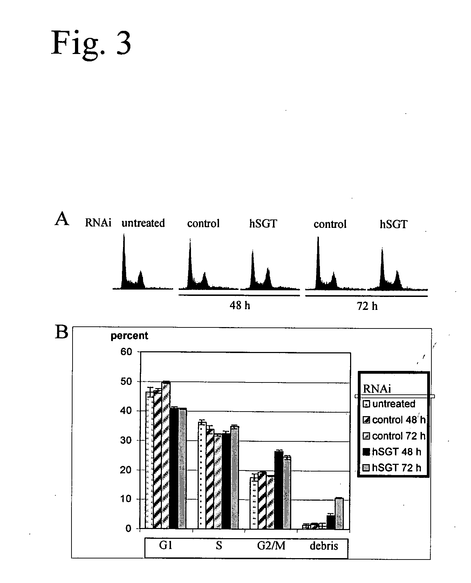

To understand why the proliferation of hSGT knock down cell populations was reduced, cell cycle distribution was analyzed by FACS (Fig. 3 A). At 48 h and 72 h after transfection, the G1 fraction of hSGT-depleted cell populations was reduced compared to controls, whereas the G2/M fraction was increased. In contrast, the percentage of cells in S phase was barely altered, which was independently confirmed through determination of the number of BrdU-incorporating cells in populations with or without reduced levels of hSGT (data not shown).

While proliferation of hSGT-depleted populations declined continuously up to 72 h after transfection (Fig. 2 C), the accumulation of cells in G2/M did not continuously increase over time in these populations. Instead, the increase in the G2/M-fraction was more pronounced at 48 h than at 72 h after transfection (Fig. 3 B).

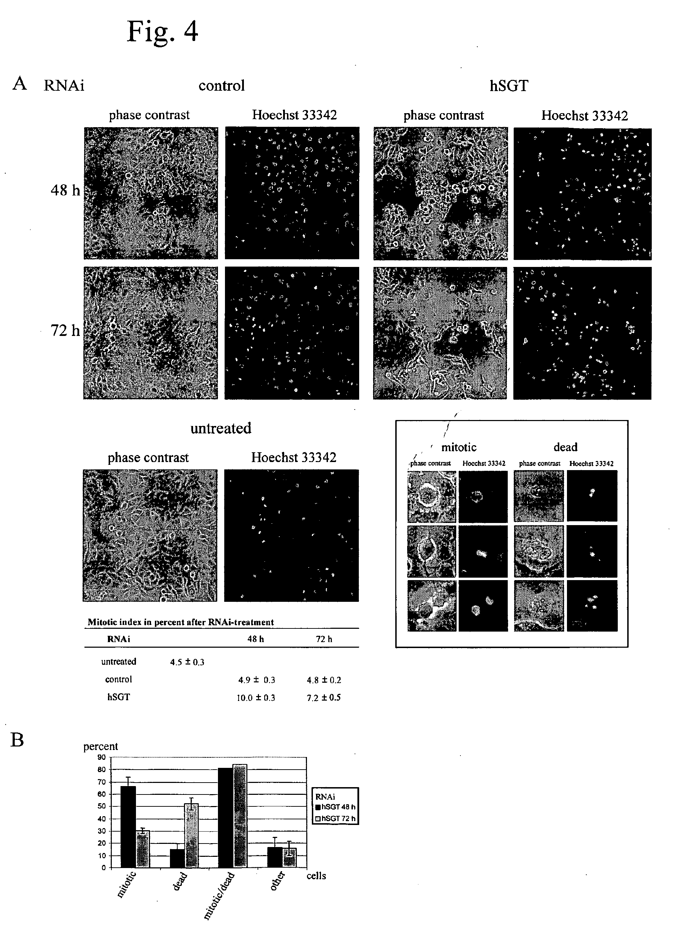

To clarify whether M-phase was affected after hSGT depletion, the mitotic index was determined in depleted and control populations after staining of the cells with Hoechst 33342 (Fig. 4 A). At 48 h after transfection the mitotic index had doubled in the hSGT-depleted cell populations (10% +/- 0.3) compared to controls (4.9% +/- 0.3), while at 72 h after transfection the mitotic index in the hSGT-depleted culture was still elevated (7.2% +/-0.5) compared to controls but lower than the value determined at 48 h after transfection with *hsgt*-specific siRNA. This significant increase in the mitotic index, observed in hSGT-depleted cell populations clearly suggested an arrest in mitosis as one possible reason for the increase in the G2/M fraction size, decrease in the G1-fraction size, and growth retardation observed in hSGT knock down populations.

The observed changes in the mitotic index of hSGT-depleted cell populations were in good agreement with the results obtained through FACS-analysis, which had shown that the G2/M fraction of the hSGT-depleted cultures also peaked around 48 h after transfection (Fig. 3). A possible explanation for this became apparent when the fraction of mitotic and dead cells was determined in the non-adherent fraction of hSGT-depleted cultures (Fig. 4 B). While the majority of the non-attached cells accumulated in M-phase at 48 h after transfection, a higher proportion of dead cells was detected at 72 h. When the number of mitotic and dead cells was combined, they added up to similar values at each time point. Therefore, the time-dependent change in the ratio between mitotic and dead cells in the non-adherent fraction (Fig. 4 B), pointed to the possibility that mitotic arrest was frequently followed by cell death. This would clearly offer a good explanation for the complex and time-dependent cell cycle distribution of hSGT-depleted cell populations (Fig. 3) as well as their continuously reduced growth (Fig. 2 C).

To test if the integrity of microtubule-based structures was affected through hSGT knock down, immunofluorescence analysis was performed using an α-tubulin-specific antibody and confocal laser microscopy. Analysis of cells from the adherent fraction of hSGT-depleted populations revealed no significant alterations in their α-tubulin staining pattern when compared to control treated cultures (Fig. 5 A). However, comparison of control and hSGT knock down cultures revealed two differences: (i) hardly any mitotic cell could be detected in the adherent fraction of hSGT-depleted cell populations, and (ii) this fraction appeared to contain more binucleated cells. Therefore, the proportion of binucleated cells in the total hSGT-depleted cell population (adherent and non-adherent) was subsequently determined. Compared to controls which contained 2% (+/- 0.2) binucleated cells, hSGT-depleted populations contained 5,7% (+/- 0.4) at 48 h and 3,6% (+/- 0.3) at 72 h after transfection. This suggested that in addition to the increased mitotic index, enhanced formation of binucleated cells might have also contributed to the reduction in G1- and the increase in G2/M-fraction size observed after hSGT depletion.

When the non-adherent fraction of hSGT-depleted cell populations was subjected to immunofluorescence analysis at 72 h after transfection, a high proportion of cells with mitotic structures was detected (Fig. 5 B, a). Several cells contained one complete spindle (Fig. 5 B, b), whereas others contained structures resembling tetra-polar spindles (Fig. 5 B, c). These cells were speculated to represent binucleated cells after entry into mitosis. Identically processed samples from populations treated with control siRNA showed hardly any cells (Fig. 5 B, d), demonstrating that mitotic cells could not be collected from control populations through the gentle aspiration applied and therefore proving that the transfection procedure itself was not able to induce a detachment of mitotic cells. Therefore detachment of mitotic cell was specific to hSGT-depleted populations.

### Example 11: hSGT depletion induced defects in cell division

To investigate how hSGT depletion affected progress through mitosis, time-lapse video microscopy was performed. First, it was determined that cells from control-treated populations required approximately 2 h to complete cell division (Fig. 6 A), and therefore behaved like untreated NBE cells. In contrast, at least 60% of the dividing cells from hSGT-depleted populations monitored at time intervals between 30 h and 90 h after siRNA treatment showed clear defects in cell division (Fig. 6 B-E). The percentage of affected cells clearly increased up to 80% towards later time points after siRNA treatment (between 72 h and 90 h). For the majority of these cells, arrest appeared to occur in prometa- or metaphase, based on the fact that these cells were perfectly round for up to 12 h while structures resembling a metaphase plate were detectable (Fig. 6 B-E). Since detection of a metaphase plate is not necessarily affected by single unattached chromosomes, no further distinction between prometa- and metaphase could be made at this point. A significant proportion of cells from hSGT-depleted populations appeared to stay arrested in mitosis until the end of the particular time interval monitored (Fig. 6 B). The fact that cells from the non-adherent fraction of hSGT knock down populations could not be cultivated beyond 94 h after siRNA treatment (data not shown), suggested that not many of these cells were able to re-enter the cell cycle. In agreement with this assumption was the observation that many cells from hSGT-depleted populations died after arrest in mitosis. In most of the cases, massive membrane alteration preceded cell death (Fig. 6 C).

Apart from the main phenotype (mitotic arrest followed by cell death) described above, a significant proportion of cells were observed that required up to 12 h to progress through mitosis and died afterwards apparently in early G1 clearly before midbody abscission (Fig. 6 D). Another minor fraction of cells could not complete cytokinesis after prolonged mitosis and became binuclear (Fig. 6 E). This latter observation strongly supported the notion that defects in the completion of cytokinesis was responsible for the hSGT-depletion dependent rise in binucleated cells.

The results obtained through time-lapse video microscopy revealed that arrest in mitosis was a major cause for the observed increase of the mitotic index in hSGT-depleted cell populations. It is important to note that the frequency of cell death observed after mitotic arrest was significantly higher around 72 h after siRNA treatment than around 48 h, suggesting that death after mitotic arrest had prevented the continuous accumulation of mitotic cells in hSGT knock down populations.

### Example 12: hSGT localized in cell division-relevant structures

To identify possible sites of action for hSGT protein, the subcellular localization of this protein was examined using confocal laser scanning microscopy in dividing cells from asynchronously growing human NBK cultures. In prophase and anaphase A, generally no specific accumulation of hSGT protein could be observed in any particular structure (Fig. 7 A and C), while in metaphase a weak signal was detected at the spindle poles (Fig. 7 B, arrows). In anaphase B and early telophase, specific accumulation of hSGT protein was detected in the region between the chromosomes (Fig. 7 D-F, arrows) while during late telophase and up to early G1, this protein was detected in structures resembling the midbody (Fig. 7 G and H, arrows).

In order to verify whether hSGT protein accumulated in the midzone - a microtubule-based structure which is important for spindle elongation -, and later on in the midbody, cells were co-stained for hSGT protein and α-tubulin. Thereby, it was shown that the midzone-specific α-tubulin signal observed in anaphase B coincided with major site of hSGT protein accumulation (Fig. 8 A-D), indicating that hSGT indeed localized in the midzone during this stage of mitosis. In contrast to the α-tubulin-specific staining in late telophase which extended into the cytoplasm of both daughter cells, hSGT protein accumulation appeared to be restricted to the midbody (Fig. 8 E-H). In early G1-phase, both hSGT- and α-tubulin-specific signals were detected in the midbody but independent α-tubulin-specific signals could also be detected at other sites (Fig. 8 I-J).

The midzone and the midbody are related and cell division-relevant structures. The fact that hSGT accumulated in these structures in itself supports the idea that hSGT could play a role in cell division and is therefore in good agreement with results obtained from the hSGT knock down experiments. These localization results furthermore pointed to relevant structures where hSGT could possibly act during cell division.

### Example 13: hSGT protein as detected by Western blotting showed a mitosis-specific migration pattern in SDS-PAGE and two-dimensional gel electrophoresis

Western blot analysis of hSGT from cells at different phases of the cell cycle revealed a slightly altered pattern for hSGT protein from the M/G1 fraction (Fig. 1 B). To test if this difference was M-phase specific, cells from this phase of the cell cycle were purified through mitotic shake-off and subsequently subjected to SDS-PAGE and Western blotting. Results obtained showed that essentially the same three fractions with apparent molecular weights of 36, 38 and 39 kDa could be distinguished when hSGT from mitotic and interphase cells were analyzed, however, with variations in signal intensities (Fig. 9 A). Most strikingly, the 39 kDa fraction appeared more pronounced in the case of the interphase cell extracts while the 38 kDa fraction was more pronounced in the mitotic cells extracts. A more detailed analysis was carried out using two dimensional gel electrophoresis (2D). After 2D analysis of extracts from interphase and mitotic cells, the three hSGT fractions observed in SDS-PAGE could be resolved into at least 10 separate polypeptide species, as detected by Western blotting (Fig. 9 A). This 2D analysis clearly demonstrated several differences in the abundance of distinct polypeptide species between mitotic and interphase cell extracts. Some hSGT-polypeptides were more abundant either in interphase or in mitotic cell extracts (Fig. 9 A, open arrow heads), while other polypeptide species were only detectable in interphase extracts (open arrows). At least two polypeptide species appeared to be exclusively detectable in mitotic extracts (filled arrows), and could therefore represent mitosis-specific hSGT polypeptide species.

Protein phosphorylation is a key regulatory event during mitosis, and evidence that hSGT is a phospho-protein was reported previously. In order to obtain first indications whether mitosis-specific migration of hSGT protein observed after SDS-PAGE and Western blotting was due to differential phosphorylation, alkaline phosphatase treatment was performed prior to Western blot analysis of extracts from mitotic cells. Alkaline phosphatase treatment almost caused a complete shift of all higher molecular range hSGT polypeptides from mitotic extracts (apparent molecular weight of 38 and 39 kDa) to the lower molecular weight range (36 kDa) (Fig. 9 B). This indicated that phosphorylation at serine and/or threonine residues could at least in part account for the occurrence of hSGT polypeptides with apparent molecular weights higher than 36 kDa and thereby also for the mitosis-specific migration pattern of hSGT protein.

## Claims

1. An in vitro method for inhibiting the propagation of an undesired cell population, the method comprising
(i) introducing an antagonist of SGT into at least one cell of said cell population,
and
(ii) cultivating said cell population for a time period sufficient to allow said SGT antagonist to be effective, thereby inactivating and/or depleting SGT in said cell population.

2. The method according to claim 1, wherein the cell population is in the mitotic stage.

3. The method according to claim 1, wherein the cell population is in a resting stage.

4. The method according to any of claims 1 to 3, wherein the cell population is a population of human cells.

5. The method according to any of claims 1 to 4, wherein the SGT antagonist is selected from the group consisting of a SGT-specific siRNA, a transcriptional regulator of the SGT gene, a SGT gene antisense molecule, a SGT mRNA specific ribozyme, an antibody against a SGT polypeptide, a SGT-specific aptamer and a SGT-specific mutein.

6. The method according to claim 5, wherein the SGT antagonist is a SGT-specific siRNA.

7. The method according to claim 6, wherein the siRNA comprises a sequence as defined by SEQ ID NOs:3 and/or 4.

8. Use of an SGT antagonist for the manufacture of a medicament for the treatment of a disease which is caused by the propagation of an undesired cell population.

9. The use according to claim 8, wherein the disease which is caused by the propagation of an undesired cell population is a cancer disease.

10. The use according to claim 9, wherein the cancer disease is selected from the group consisting of neuroblastoma, intestine carcinoma, rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroid carcinoma, papillary thyroid carcinoma, follicular thyroid carcinoma, anaplastic thyroid carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors, glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma and plasmocytoma.

11. The use according to claim 10, wherein the cancer disease is cervical carcinoma, neuroblastoma, glioblastoma and/or breast carcinoma.

12. The use according to any of claims 8 to 11, wherein the SGT antagonist is a SGT-specific siRNA.

13. A medicament containing a SGT antagonist, optionally together with a pharmaceutically acceptable carrier, for the treatment of a disease caused by the propagation of an undesired cell population.

14. The medicament according to claim 13, wherein the disease is a cancer disease.

15. A method for screening candidate compounds for at least one SGT antagonist with the ability to inhibit the propagation of a cell population, the method comprising the following steps:
(i) contacting a cell population with a candidate compound, thereby enabling the introduction of said candidate compound into the cells of said cell population,
(ii) cultivating said cell population for a time period sufficient to allow the candidate compound to be effective, and parallel cultivating a control cell population which has not been contacted with the candidate compound,
and
(iii) monitoring cell growth and/or cell properties in said cell population and in the control cell population,
wherein a reduced growth and/or altered cell properties as compared to the control cell population is indicative that the candidate compound is an SGT antagonist which inhibits the propagation of a cell population.

16. The method according to claim 15, the method comprising the additional steps:
(iv) qualitatively and/or quantitatively detecting SGT expression levels in said cell population and in the control cell population, wherein a lower level of SGT expression is indicative of a compound that is a SGT antagonist,
and
(v) determining whether a lower level of SGT expression correlates with a reduced growth and/or altered cell properties of the cell population being contacted with the candidate compound.

17. The method according to claim 15 or 16, wherein the cell population is in the mitotic stage.

18. The method according to any of claims 15 to 17, wherein the cell population is a population of human cells.

19. A method for the preparation of a pharmaceutical composition wherein a SGT antagonist inhibiting the propagation of an undesired cell population is identified according to any of claims 14 to 17, synthesized in adequate amounts, and formulated into a pharmaceutical composition.
